# EUROPEAN PATENT SPECIFICATION

(11) **EP 0 324 867 B1**
(45) Date of publication and mention of the grant of the patent: **11.08.1993**
(21) Application number: 88100628.2
(22) Date of filing: 18.01.1988
(51) Int. Cl.: C07K 15/04, C12P 21/00, C12N 1/20

(54) **Fc-binding protein and strain of producing the same**
Fc-bindendes Protein und Stamm zu dessen Herstellung
Protéine pouvant se lier à Fc, et souche pour la produire

(43) Date of publication of application: 26.07.1989
(73) Proprietor: MERCIAN CORPORATION, Chuo-ku, Tokyo (JP)
(72) Inventor: Kaneto, Rei, Yokohama-shi Kanagawa-ken (JP); Okamura, Kazuhiko, Fujisawa-shi Kanagawa-ken (JP); Ishikura, Tomoyuki, Chigasaki-shi Kanagawa-ken (JP)
(74) Representative: Müller-Boré & Partner Patentanwälte

(56) References cited:
- EP-A- 0 107 509
- MICROBIOL. IMMUNOL., vol. 23, no. 2, 1979, pages 51-60; J. MOVITZ et al.: "Physico- and immunochemical properties of staphylococcal protein. A extracellularly produced by a set of mutants from Staphylococcus aureus cowan I"
- EUR. J. BIOCHEM., vol. 78, 1977, pages 471-490; J. SIÖDAHL: Structural studies on the four repetitive Fc-binding regions in protein A from Staphylococcus aureus"
- PROC. NATL. ACAD. SCI. USA, vol. 80, February 1983, pages 697-701; S. LÖFDAHL et al.: "Gene for Staphylococcal protein A"

## Description

### FIELD OF THE INVENTION

The present invention relates to a protein having an ability of binding with the Fc part of immunoglobulin G (hereinafter referred to as "IgG") and also to microorganisms of secreting and producing the said protein.

### BACKGROUND OF THE INVENTION

Substances of strongly and specifically binding with the Fc part of IgG can effectively be used in purification or concentration of antibody, and these are now especially noticed as useful in the field of clinical diagnostics and biochemical studies. For instance, the most typical substance is protein A, and further, the protein A as fixed to an insoluble carrier can also be applied to plasma perfusion so as to remove the immune complex precipitated, in addition to the above-mentioned use, and hence this can be used for the purpose of therapy of cancers or autoimmune diseases (e.g., Cancer, 46, 675 (1980)).

The protein A is eluted from cell walls of Staphylococcus aureus, Cowan I strain with an enzyme lysostaphin and is isolated and purified by means of various kinds of column chromatography (Eur. J. Biochem., 29, 572 (1972)). In addition, production of the protein A from a strain of Staphylococcus aureus with high methicillin resistance by extracellular secretion has been tried (ibid., 174, 623 (1977)).

On the other hand, it is known that a mutant strain derived from Staphylococcus aureus by UV-irradiation can secrete and produce several kinds of substances (for example, having molecular weight of 17,000, 57,000, etc.) which have an ability of binding with the Fc part of IgG similarly to the protein A, although having a smaller molecular weight than the protein A (Microbiol. Immunol., 23, 51 (1979)).

According to the above-mentioned prior art, although the production of the protein A and its analogue compounds which are usable as a clinical diagnosticum or the like is possible, the means of eluting the same from cell walls is complicated and the use of some expensive enzymes is indispensable in the elution means. In addition, although the secretory production from the strain with high methicillin resistance is excellent in that this does not require the treatment of cell walls, some careful attention is required to be paid to the treatment of the strain itself since the strain has a resistance also to important medicines such as penicillin G, sulfonamide drugs, tetracycline, erythromycin, cephalosporin, ampicillin, etc.

Moreover, the method of producing protein A- analogue compounds is defective in that this require the isolation and recovery of a desired single substance from a mixture containing several kinds of substances with different molecular weights.

### SUMMARY OF THE INVENTION

The present inventors treated Staphylococcus aureus, Cowan I strain, which can be treated relatively easily, for mutation to investigate the productivity of protein A- analogue substances, and as a result, have found that the mutant derived from the said strain by mutation treatment with N-methyl-N′-nitro-N-nitroguanidine (hereinafter referred to as "NTG") can produce a new single substance which is not disclosed in prior literatures and which has a protein A-like property, and hence have achieved the present invention.

Accordingly, the first object of the present invention is to provide a protein which has an ability of binding with the Fc part of immunoglobulin G and which has the following properties:
(A) Molecular weight: 24,000
(B) Isoelectric point: pH 4.5
(C) Ultraviolet absorption: maximum absorption at 275nm (E^{1%}_{1cm} = 1.60)
(D) Binding ability with human IgG: 11mg/mg
The protein is hereinafter referred to as "SRP-2".

The second object of the present invention is to provide Staphylococcus aureus SR-1 having an ability of producing the said protein by extracellular secretion.

### BRIEF DESCRIPTION OF DRAWING

The drawing attached hereto shows the ultraviolet absorption spectrum of SRP-2 substance of the present invention.

### DETAILED DESCRIPTION OF THE INVENTION

The Fc part of immunoglobulin G (IgG) as herein referred to is a region of IgG which is derived from mammals such as human, monkey, rabbit, guinea pig, dog, pig, mouse, etc. and which is one kind of per se known antibodies, and the said region is outside the Fab part of IgG containing the antigen-binding region (V domain) and is able to bind with a cell membrane. (For example, refer to Metabolism and Disease, 16, 1881 (1979).)

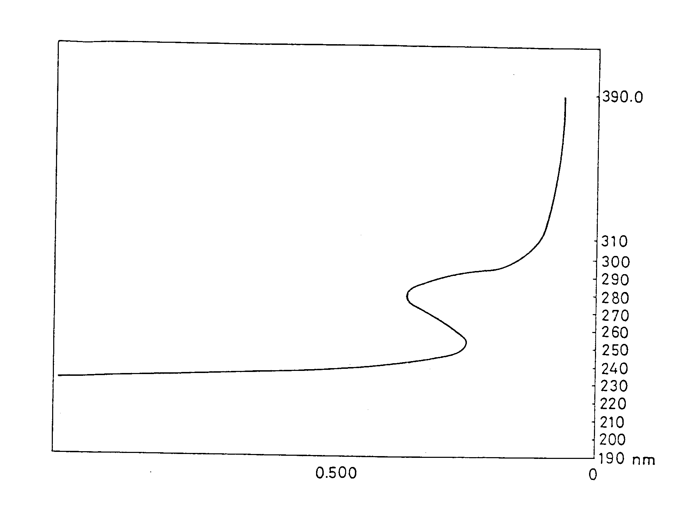

The properties of the SRP-2 were measured by means of the following methods.
(i) The molecular weight was measured by a so-called SDS-gel electrophoresis method in accordance with Laemmli et al's method (Nature, 227, 1970), where a protein molecule marker (manufactured by Sigma Co.) was used and, after strained with Coomassie Blue G 250, the molecular weight of the SRP-2 was measured.
(ii)The isoelectric point was measured by means of an isoelectric point electrophoresis method in accordance with Reisner et al's method (Anal. Biochem., 64, 509 (1975)), where the gel was, after migrated, put on a 1% swine serum-added agarose plate and kept at 20°C for 24 hours, and then the position of the SRP-2 was detected from the precipitation zone whereby the position thus detected was compared with the isoelectric point marker to obtain the isoelectric point of the present SRP-2.
(iii) For the determination of the ultraviolet absorption, the SRP-2 was dissolved in distilled water and the ultraviolet absorption spectrum was obtained, from which the wavelength of the maximum absorption zone and the value of E^{1%}_{1cm} were calculated. (Refer to Fig. 1 as attached hereto.)
(iv)The binding ability with IgG was measured by means of Becker's method (Immunochem., 6, 539 (1969)), where the binding ability of the present SRP-2 with human immuno-globulin G was measured and was designated by the weight of the human immunoglobulin G as bound with the unit weight (1mg) of the present SRP-2. In addition, the binding ability of protein A was also measured and was 8mg, which apparently shows that the binding ability of the present substance SRP-2 per unit amount is larger than that of protein A.
(v) For reference, the present SRP-2 was decomposed with 6N HCl for 24 hours at 110°C and the decomposed product was analyzed with an automatic amino acid-analyzer (Hitachi 835 Type), and the result is shown in the following Table.

**Table**

| Amino Acid Composition | |
|---|---|
| Amino Acid | Number of Residues |
| Aspartic Acid | 45 |
| Threonine | 2 |
| Serine | 15 |
| Glutamic Acid | 43 |
| Glycine | 5 |
| Alanine | 22 |
| Valine | 2 |
| Isoleucine | 9 |
| Leucine | 23 |
| Tyrosine | 4 |
| Phenylalanine | 12 |
| Lysine | 21 |
| Histidine | 2 |
| Arginine | 3 |
| Proline | 12 |
| Methionine | 2 |

The present substance SRP-2 which is specified by the above-mentioned characteristics can be obtained by incubating Staphylococcus aureus SR-1 strain in a nutrient medium, whereupon the cultured solution is subjected to a per se known purification treatment including ultrafiltration, column chromatography and dialysis so as to isolate and purify the present substance SRP-2.

Staphylococcus aureus SR-1 strain is isolated by mutation of the parent strain Staphylococcus aureus Cowan I (see Example 1, below).

The parent strain Staphylococcus aureus, Cowan I has been deposited at American Type Culture Collection under (ATCC 12598).

Staphylococcus aureus SR-1 thus obtained, which is one mutant strain having the SRP-2 producing ability, has been deposited at Fermentation Research Institute Agency of Industrial Science and Technology in Japan on December 22, 1987.

The microbiological properties of the said mutant strain Staphylococcus aureus SR-1 are same as those of the parent strain Staphylococcus aureus, Cowan I, except that the former has the SRP-2 producing ability. (For example, refer to J. Path. Bact., 48, 169 (1939).)

### Example 1

### Preparation of Staphylococcus aureus SR-1 Mutation Process

One loopful amount from slant culture (common agar medium) of Staphylococcus aureus, Cowan I strain was inoculated in Todd-Hewitt medium (20ml/250ml, Erlenmeyer flask) and incubated at 37°C for 20 hours under shake.

5ml was taken out therefrom and inoculated into a 250ml Erlenmeyer flask containing 50ml of the same medium and again incubated at 37°C for 2 hours under shake. The bacteria thus incubated were harvested by centrifugation and suspended in 10ml of 0.05M phosphate buffer (pH 6), and NTG was added thereto in the final concentration of 100µg/ml and treated at 37°C for 30 minutes. The bacteria thus treated were harvested by centrifugation, washed with the same phosphate buffer and again centrifuged. Lastly, the bacteria harvested were suspended in 10ml of phosphate buffer, and 0.05ml of the resulting suspension was spread over Todd-Hewitt agar medium containing 5µg/ml of streptomycin. After incubated at 37°C for 24 hours, the colonies formed were isolated and strains with high secretory producibility were screened by the following method.

### Screening of Strains with High Secretory Producibility

The colonies obtained in the above-mentioned process were inoculated in Todd-Hewitt agar medium to which 1% of swine serum had been added and incubated at 37°C for 24 hours. Next, the medium was transferred to 20°C-incubator and kept for 24 hours at said temperature, and then the size of the precipitation zones as generated around the colonies were compared and the colony with the largest sedimentation zone was selected, which was designated Staphylococcus aureus SR-1 strain.

### EXAMPLE 2

### Production of SRP-2 Substance

One loopful amount of common agar medium-cultured bacteria of SR-1 strain was inoculated in G-medium (composed of 4% peptone, 1% yeast extract and 1% lactic acid, pH 7, 50ml/250ml Erlenmeyer flask) and incubated at 37°C for 24 hours under shake. 0.1ml was taken out therefrom and inoculated in 10 Erlenmeyer flasks with stainless steel coil each containing 100 ml of the same medium, and then incubated at 37°C for 24 hours. The solutions incubated were combined and filtered in an ultrafiltration apparatus Minitan (manufactured by Milli Pore Japan Co., with a filter membrane of fractionation molecular weight 100,000). 40g of salt was added to 650ml of the resulting filtrate and the active fraction was isolated by hydrophobic chromatography. After being freeze-dried, 5ml of the resulting product was dissolved in 5ml of 0.05M tris-buffer (pH 7.5) and purified by gel-filtration (with Sephadex G-100). After dialysis, the product thus purified was again freeze-dried, whereby 7mg of a white powder of SRP-2 was obtained.

As apparent from the above description, the SRP-2 substance of the present invention has an ability of binding with IgG and can specifically bind with the Fc part of IgG similarly to protein A, while the binding force of the present SRP-2 substance per unit amount is higher than that of protein A. Thus, the excellent effect of the present invention is apparent.

## Claims

1. A protein with the ability of binding the Fc part of immunoglobulin G, produced by Staphylococcus aureus deposited at Fermentation Research Institute Agency of Industrial Science and Technology in Japan under the deposition number FERM BP-1632.

2. Staphylococcus aureus accoring to Claim 1, deposited at Fermentation Research Institute Agency of Industrial Science and Technology in Japan under the deposition number FERM BP-1632.

## Patentansprüche

1. Protein mit der Bindungsfähigkeit des Fc-Teils von Immunglobulin G, produziert durch den bei Fermentation Research Institute Agency of Industrial Science and Technology in Japan unter der Hinterlegungs-Nr. FERM BP-1632 hinterlegten Staphylococcus aureus.

2. Staphylococcus aureus nach Anspruch 1, hinterlegt bei Fermentation Research Institute Agency of Industrial Science and Technology in Japan unter der Hinterlegungs-Nr. FERM BP-1632.

## Revendications

1. Protéine capable de se lier à la partie Fc d'une immunoglobuline G, produite au moyen de Staphylococcus aureus déposé auprès de l'Institut de Recherche sur la Fermentation, Agence des Sciences Industrielles et de la Technologie (Fermentation Research Institute Agency of Industrial Science and Technology), Japon, sous le numéro d'enregistrement FERM BP-1632.

2. Staphylococcus aureus selon la revendication 1, déposé auprès de l'Institut de Recherche sur la Fermentation, Agence des Sciences Industrielles et de la Technologie, Japon, sous le numéro d'enregistrement FERM BP-1632.
